# EUROPÄISCHE PATENTANMELDUNG

(11) **EP 4 252 692 A1**
(43) Veröffentlichungstag der Anmeldung: **04.10.2023**
(21) Anmeldenummer: 22166100.2
(22) Anmeldetag: 31.03.2022
(51) Int. Cl.: A61B 18/14, A61B 18/00, A61B 90/00

(54) **ELEKTROCHIRURGISCHES INSTRUMENT MIT PERMANENTSPANNER**

(71) Anmelder: Erbe Elektromedizin GmbH, 72072 Tübingen (DE)
(72) Erfinder: KARWEI, Dietmar, 72131 Ofterdingen (DE)
(74) Vertreter: Rüger Abel Patentanwälte PartGmbB

(57) **Zusammenfassung**

Das erfindungsgemäße elektrochirurgische Instrument (10) weist eine Elektrode (22) und eine Isolatoranordnung auf. Diese umfasst einen Instrumentenschlauch (12) und eine an seinem distalen Ende angeordnete Isolatoranordnung. In Instrumentenschlauch (12) ist ein längliches Schubelement (14) zum Festlegen der Position der Elektrode (22) relativ zum distalen Ende der Isolatoranordnung angeordnet. Das Instrument (10) weist einen Anschlag (35) und einen Gegenanschlag (37) auf, wobei der Gegenanschlag der Elektrode (22) und/oder dem Schubelements (14) zugeordnet ist. Eine elastisch verformbare Einrichtung (62) ist dazu bestimmt, den Gegenanschlag (37) in distale Richtung gegen den Anschlag (35) vorzuspannen, um beim Abknicken oder Krümmen des Instrumentenschlauchs (12) eine Bewegung der Elektrode (22) in proximale Richtung relativ zu dem distalen Ende (20) der Isolatoranordnung und/oder des Instrumentenschlauchs (12) zu verhindern.

## Beschreibung

Die Erfindung betrifft ein Instrument mit einem Schlauch, in welchem sich ein längliches Schubelement erstreckt, welches mit einer Elektrode am distalen Ende des Schubelements gekoppelt ist.

Aus der DE 10 2006 006 052 A1 ist ein Behandlungsgerät für ein Endoskop bekannt, welches eine flexible Hülle aufweist, die in einen Einführkanal eines Endoskops einführbar ist und einen elektrisch leitenden Betätigungsdraht aufweist, der in der flexiblen Hülle vorschiebbar und zurückziehbar ist. Die Elektrode soll unabhängig von dem Betätigungsdraht von dem Hochfrequenzbehandlungsgerät lösbar sein. In dem Behandlungsgerät kann eine Feder angeordnet sein, die auf die Elektrode eine Expansionskraft ausübt, die in Richtung des proximalen Endes der Elektrode wirkt. Die Elektrode lässt sich mit Hilfe des Betätigungsdrahts durch ein Loch in dem distalen Ende des Behandlungsgeräts schieben, so dass die Elektrode ein Stück über das distale Ende der Hülle aus dem Loch übersteht.

Bei derartigen Instrumenten kann die Länge des Stücks, das über das distale Ende der flexiblen Hülle übersteht, von der Krümmung oder Abknickung der Hülle abhängen. Dies kann bei einigen Instrumenten auf einen Spalt zurückgeführt werden, der zwischen der fixierten flexiblen Hülle und dem Betätigungsdraht freigelassen ist. Der Spalt kann beispielsweise durch Durchmessertoleranzen der Bauteile entstehen und kann auch bereitgestellt werden, um ein für die Aus- und Einfahrbewegung notwendiges Spiel zwischen den Bauteilen bereitzustellen. Im Übrigen wird für die flexible Hülle eines derartigen Instruments in der Regel Kunststoff als Material verwendet, das sich unter Krafteinwirkung verformen kann. Dies führt dazu, dass wenn die Elektrodenspitze in der neutralen gestreckten Lage des Instruments ein bestimmtes Stück über das distale Ende der Hülle übersteht, bei einem abgewinkelten oder gekrümmten Instrument nicht mehr garantiert werden kann, dass die Elektrodenspitze immer noch diesen Abstand von dem distalen Ende der Hülle aufweist.

In der WO 2009/030 324 A1 wird diesem Umstand Rechnung getragen, indem am Ende des Schutzschlauches des Instruments ein Elastomerring angeordnet ist, welcher die Nadelelektrode umgibt. Der Elastomerring ist derart ausgebildet, dass er eine definierte Haltekraft induziert, die die Nadelelektrode in der vorab eingestellten Position festsetzt. Beim Vor- und Zurückschieben der Nadelelektrode muss diese Reibungskraft zum Festsetzen der Nadelelektrode jedoch stets überwunden werden.

Aufgabe der Erfindung ist es, ein Instrument mit einem verbesserten Konzept, insbesondere zum Einhalten eines Abstands einer Elektrodenspitze vom distalen Ende eines Isolators anzugeben.

Diese Aufgabe wird mit einem elektrochirurgischen Instrument nach Anspruch 1 gelöst:

Das erfindungsgemäße elektrochirurgische Instrument weist eine Elektrode, beispielsweise aus Wolfram, und eine Isolatoranordnung auf, welche einen Instrumentenschlauch und ein Endstück aufweisen kann. Das Endstück kann ein aus Keramik bestehender Isolatorkörper sein. In dem Instrumentenschlauch ist ein längliches Schubelement (z.B. ein Innenschlauch oder Draht) zum Verändern der Position der Elektrode, insbesondere der Elektrodenspitze relativ zum distalen Ende der Isolatoranordnung, beispielsweise zum Vorschieben der Elektrode über das Ende der Isolatoranordnung hinaus, angeordnet.

Das Instrument weist einen Anschlag auf, der z.B. in oder an dem Isolatorkörper ausgebildet sein kann. Zudem weist das Instrument einen Gegenanschlag der Elektrode oder des Schubelements auf. Eine elastisch verformbare Einrichtung, z.B. ein federndes Element (Federelement), ist angeordnet und eingerichtet, den Gegenanschlag in distaler Richtung gegen den Anschlag vorzuspannen, um beim Abknicken oder Krümmen des Schlauches die Position der Elektrode, insbesondere die Position der Elektrodenspitze bezüglich des distalen Endes der Isolatoranordnung und/oder bezüglich des distalen Endes des Schlauches zu halten bzw. festzulegen. Eine unerwünschte Bewegung der Elektrode in proximaler Richtung, insbesondere eine Bewegung der Elektrodenspitze in proximaler Richtung relativ zu dem distalen Ende der Isolatoranordnung und/oder des Instrumentenschlauchs auch bei starkem Abknicken oder Krümmen des Instrumentenschlauchs, wird damit wirksam unterbunden.

Das Schubelement erstreckt sich von dem distalen Ende des Instruments bis zu seinem proximalen Ende, so dass die Bewegung der Elektrode durch Einwirkung auf das proximale Ende des Instruments möglich ist. Das Schubelement kann ein mit der Elektrode elektrisch verbundener elektrischer Leiter zum Beaufschlagen der Elektrode mit elektrischer Hochfrequenzleistung sein. Die Elektrode kann beispielsweise zum Schneiden von biologischem Gewebe genutzt werden. Das Schubelement kann auch ein schubsteifer Innenschlauch sein, der in dem Lumen des äußeren Instrumentenschlauchs angeordnet ist. In oder an diesem schubsteifen inneren Schlauch kann wiederum ein elektrischer Leiter, z.B. ein Draht, angeordnet sein, der sich durch den inneren Schlauch erstreckt und der Stromversorgung der Elektrode dient.

Der äußere Instrumentenschlauch ist aus elektrisch isolierendem Material ausgebildet, beispielsweise aus Kunststoff. Vorzugsweise ist auch der innere Schlauch aus Kunststoff.

Der Außendurchmesser des Schubelements ist vorzugsweise kleiner als der Innendurchmesser des Instrumentenschlauchs, um ein Querspiel zum weitgehend kräftefreien Vorschieben und vorzugsweise Zurückziehen des Schubelements in dem Instrumentenschlauch bereitzustellen.

Der Anschlag ist bevorzugt relativ zu dem distalen Ende der Isolatoranordnung und/oder dem distalen Ende des Instrumentenschlauchs fixiert. Der Anschlag legt eine Position des gegen den Anschlag vorgespannten Gegenanschlags relativ zu dem Ende der Isolatoranordnung und/oder relativ zu dem Instrumentenschlauch fest.

Der Gegenanschlag ist mit der Elektrode und/oder dem Schubelement gekoppelt, insbesondere an der Elektrode und/oder dem Schubelement fixiert oder ausgebildet. Der Gegenanschlag und die Elektrode und/oder das Schubelement sind zueinander unbeweglich. Der Gegenanschlag kann an der Elektrode, einem Sockelelement oder dem Schubelement ausgebildet, insbesondere angeformt, sein. Der Gegenanschlag kann auch durch ein gesondertes Element gebildet sein, welches an der Elektrode, einem Sockelelement oder dem Schubelement befestigt ist.

Bei der elastisch verformbaren Einrichtung kann es sich um das Schubelement oder einen Abschnitt desselben handeln, welcher dazu eingerichtet, insbesondere geformt, und bestimmt ist, beim Vorschieben der Elektrode elastisch verformt zu werden, um den Gegenanschlag in distale Richtung gegen den Anschlag vorzuspannen. Alternativ oder zusätzlich kann die elastisch verformbare Einrichtung ein von dem Schubelement gesondertes Federelement, beispielsweise eine Druckfeder, aufweisen oder sein. Die elastisch verformbare Einrichtung bildet eine elastische Längenreserve (Längenspeicher). Auf Grund dieser elastischen Längenreserve kann das distale Ende der Elektrode bei allen beim chirurgischen Eingriff mit dem Instrument vorkommenden Krümmungen oder Abknickungen des Instrumentenschlauchs stets in definierter Position relativ zu dem distalen Ende des Isolatorkörpers, insbesondere in distaler Ausschubposition gehalten werden. Die Länge des Stücks der Elektrode, das über das distale Ende des Isolatorkörpers und/oder des Schlauches übersteht, ist damit unabhängig von der Krümmung oder Abknickung des Instrumentenschlauchs. Das Schubelement kann durch einen drucksteifen Draht, insbesondere einen Metalldraht gebildet sein. Dieser kann auch der Stromversorgung der Elektrode dienen. Das Schubelement kann auch durch einen drucksteifen Zusatzschlauch gebildet sein, der in dem Lumen des Instrumentenschlauchs angeordnet ist. Dieser Zusatzschlauch kann der Elektrode oder einem als Gegenanschlagelement dienenden Elektrodensockel verbunden sein. Die Elektrode und der Elektrodensockel können einen zentralen Kanal aufweisen, der über den Zusatzschlauch mit Gas oder Flüssigkeit beaufschlagbar ist.

Der Isolatorkörper bildet das distale Ende des Instruments. Vorzugsweise ist der Anschlag an oder in dem Isolatorkörper ausgebildet. Der Isolatorkörper weist vorzugsweise einen röhrchenartigen Fortsatz auf, auf dem der äußere Instrumentenschlauch gehalten ist. Der Anschlag kann an dem distalen Ende des röhrchenartigen Fortsatzes angeordnet oder durch eine Ringschulter gebildet sein, die in dem sich durch den röhrchenartigen Fortsatz erstreckenden Kanal ausgebildet ist.

Die elastisch verformbare Einrichtung, beispielsweise das Federelement, kann proximal außerhalb des Schlauches, bspw. benachbart zu diesem angeordnet sein. An das proximale Ende des Instrumentenschlauchs kann ein Handgriff anschließen. Die elastisch verformbare Einrichtung, beispielsweise das Federelement, kann in dem Handgriff angeordnet sein.

Der Anschlag ist vorzugsweise an dem distalen Ende des Instruments z.B. in oder an dem am distalen Ende angeordneten Isolatorkörpers in der Nähe der Elektrode angeordnet. Die elastisch verformbare Einrichtung ist vorzugsweise zwischen dem proximalen Betätigungsmittels und dem Anschlag wirksam. Dadurch kann jede durch Bewegung oder Biegung oder Abwinkelung des Instruments entstehende Längendifferenz zwischen dem Instrumentenschlauch und dem die Elektrode ausfahrenden Schubelement ausgeglichen werden.

Der Isolatorkörper ist vorzugsweise von dem Instrumentenschlauch gehalten. Er kann durch Reibschluss zwischen dem Anschlagelement und dem Instrumentenschlauch in diesem befestigt sein. Bevorzugt ist die Verbindung zwischen dem Isolatorkörper und dem Instrumentenschlauch nicht stoffschlüssig. Alternativ oder zusätzlich ist die Verbindung zwischen dem Isolatorkörper und dem Instrumentenschlauch vorzugsweise nicht formschlüssig, sondern bevorzugt nur reibschlüssig.

Der Isolatorkörper steht vorzugsweise über das distale Ende des Instrumentenschlauchs über und bildet vorzugsweise das distale Ende der Isolatoranordnung. Der Isolatorkörper besteht vorzugsweise aus Keramik oder einem anderen isolierenden Material.

Der Gegenanschlag kann von einem Gegenanschlagelement gebildet sein, insbesondere von dessen distalem Ende. Das Gegenanschlagelement kann ein von der Elektrode und/oder dem Schubelement gesondertes Element sein. Das Gegenanschlagelement kann der elektrischen Kontaktierung zwischen der Elektrode und dem Schubelement dienen und z.B. als Elektrodensockel ausgebildet sein. Das Gegenanschlagelement kann beispielsweise aus Edelstahl bestehen.

Das Gegenanschlagelement kann ein Ende, in dem die Elektrode steckt, und ein anderes Ende aufweisen, in dem das distale Ende des Schubelements steckt. Der Gegenanschlag kann z.B. durch eine Stirnfläche oder eine Schulterfläche des Gegenanschlagelements gebildet sein. Das Gegenanschlagelement kann beispielsweise aus Edelstahl bestehen.

Das Gegenanschlagelement kann eine mechanische schub- und zugfeste Verbindung zwischen dem Schubelement und der Elektrode erbringen, um die Elektrode mittels des Schubelements in distaler und proximaler Richtung kontrolliert bewegen zu können. Die Hülse kann die aus distalem Ende des Schlauches und proximalem Ende der Elektrode gebildete Anordnung versteifen.

Das Schubelement bildet oder enthält die Elektrodenzuleitung zur Versorgung der Elektrode mit elektrischer Leistung und ist vorzugsweise aus flexiblem Metall, beispielsweise Nitinol ausgebildet. Wenn das Schubelement aus flexiblem Metall und eine Elektrode aus Wolfram verwendet werden, kann einerseits eine hohe Flexibilität des Schubelements und andererseits eine hohe Beständigkeit der Elektrode erhalten werden. Das Schubelement aus flexiblem Metall und die Elektrode aus Wolfram können beispielsweise mittels einer Hülse verbunden sein, welche das Gegenanschlagelement bilden kann. Alternativ kann die Elektrode ein distaler Endabschnitt des Schubelements sein.

Ist das Schubelement als innerer Schlauch ausgebildet, kann dieser mit dem proximalen Ende des Sockelelements verbunden sein, das distal die Elektrode trägt. Das Sockelelement und die Elektrode können einen Durchgangskanal aufweisen, durch den ein Gas oder Flüssigkeit zu dem oder in das Gewebe gespritzt werden kann. Der Durchgangskanal kann axial und zentral angeordnet sein.

Das erfindungsgemäße Instrument kann unabhängig von der Ausbildung des Schubelements ein Verbindungselement zum Verbinden des Instrumentenschlauches mit einem Zusatzschlauch aufweisen, der als Fluidleitungsschlauch dienen kann und parallel zu dem Instrumentenschlauch geführt ist. Das Verbindungselement kann nahtlos einstückig oder mehrteilig aufgebaut sein.

Um den Instrumentenschlauch mit dem Zusatzschlauch am distalen Ende des Instruments zu verbinden, kann das Verbindungselement wenigstens zwei parallele Fortsätze aufweisen. Der Instrumentenschlauch und/oder der Zusatzschlauch können auf je einem Fortsatz des Verbindungselements stecken. Der Fortsatz für den Instrumentenschlauch kann einen Führungskanal für die Elektrode aufweisen. Der Fortsatz für den Zusatzschlauch kann einen Fluidleitungskanal aufweisen, der mit dem Zusatzschlauch fluidisch verbunden ist. Der Fluidleitungskanal kann bis zu dem distalen Ende des Verbindungselements reichen und an seinem distalen Ende einen Düsenabschnitt aufweisen.

Das Verbindungselement ist vorzugsweise steif. Vorzugsweise ist das Verbindungselement in distaler Richtung weniger nachgiebig als der Instrumentenschlauch und weniger nachgiebig als der Zusatzschlauch. Das Verbindungselement kann das Anschlagelement bilden oder aufweisen.

Bei einem mehrteiligen Aufbau des Verbindungselements (dieser kann auch als Verbindungseinrichtung bezeichnet werden) kann ein Fortsatz von einem ersten Teil und ein weiterer Fortsatz von einem zweiten Teil gebildet werden, das ein von dem ersten Teil gesondertes Teil ist. Das erste und das zweite Teil kann jeweils aus Keramik bestehen. Ein drittes Teil des Verbindungselements, welches von dem ersten Teil und/oder dem zweiten Teil gesondert ist, dient der Verbindung des ersten Teils und des zweiten Teils miteinander und vermittelt die Verbindung des Instrumentenschlauchs mit dem Zusatzschlauch. Das dritte Teil kann beispielsweise ein Blechteil, insbesondere Metallblechteil, sein.

Das Instrument kann durch einen Arbeitskanal eines Endoskops geführt sein. In Ausführungsformen mit einem Zusatzschlauch können der Instrumentenschlauch und der Zusatzschlauch durch denselben Arbeitskanal eines Endoskops oder durch je einen eigenen Arbeitskanal geführt sein.

Der Instrumentenschlauch und der Zusatzschlauch können in einer Hülle, insbesondere in einem gemeinsamen Lumen einer Hülle, angeordnet sein, welche Hülle bis zu den distalen Enden der Schläuche reichen kann. Die Hülle kann proximal an oder in einem Handgriffteil des Instruments enden. Die Hülle kann ein über die gesamte Länge der Schläuche oder die gesamte Länge zwischen Handgriffteil und distalem Ende der Schläuche durchgehender Schrumpfschlauch sein, welcher die Schläuche umgibt und auf die Schläuche aufgeschrumpft ist.

An das Schubelement kann ein Bedienteil angeschlossen sein, das mit dem Schubelement in Schubrichtung elastisch verbunden ist. Das Bedienteil kann mit dem Schubelement auch in proximaler Richtung elastisch verbunden sein, um die Elektrode unterschiedlich weit in die Isolatoranordnung zurückziehen zu können. Zur elastischen Kopplung auch in proximaler Richtung kann die elastisch verformbare Einrichtung dienen. Alternativ kann das erfindungsgemäße Instrument eine weitere elastisch verformbare Einrichtung aufweisen. Diese ist bevorzugt zwischen einem Bedienteil und dem Schubelement wirksam. Das Instrument kann derart eingerichtet sein, dass bei einer Bewegung des Bedienteils zum Zurückziehen des Schubelements bzw. der Elektrode, ein weiteres Federelement als weitere elastisch verformbare Einrichtung in proximale Richtung gegen einen Anschlag geschoben wird. Der Anschlag kann mit dem Schubelement gekoppelt sein, um die Bewegung des Bedienteils auf das Schubelement zum Zurückziehen der Elektrode zu übertragen. Das weitere Federelement sorgt dabei für einen Längenausgleich, um die Einrichtung zum Zurückziehen der Elektrode mit beispielsweise nadelförmigen und L-förmigen Elektroden nutzen zu können, welche sich unterschiedlich weit in die Isolatoranordnung zurückziehen lassen.

Weitere vorteilhafte Ausführungsformen und Merkmale des erfindungsgemäßen Instruments ergeben sich aus nachfolgender Beschreibung, den Unteransprüchen, sowie den Figuren.

Es zeigen beispielhaft:
Figur 1 - eine perspektivische Ansicht eines Beispiels eines erfindungsgemäßen Instruments,
Figur 2 - einen distalen Endabschnitt des Beispiels des erfindungsgemäßen Instruments gemäß Figur 1,
Figur 3 - den distalen Endabschnitt gemäß Figur 2 in einer Längsschnittdarstellung durch die Ebene welche durch die Mittelachse der Fluidöffnung und die Mittelachse der Elektrodenöffnung festgelegt ist,
Figur 4 - einen Längsschnitt durch den Handgriff am proximalen Ende des Beispiels des Instruments gemäß Figur 1,
Figur 5a - einen Längsschnitt durch einen Arbeitskanal eines Endoskopschafts, durch welchen sich ein Instrumentenschlauch und ein Zusatzschlauch eines Ausführungsbeispiels des erfindungsgemäßen Instruments erstrecken,
Figur 5b - einen Längsschnitt durch zwei Arbeitskanäle eines Endoskopschafts, wobei sich der Instrumentenschlauch des Ausführungsbeispiels des erfindungsgemäßen Instruments in einem Arbeitskanal und sich der Zusatzschlauch des Ausführungsbeispiels des erfindungsgemäßen Instruments in dem anderen Arbeitskanal erstreckt,
Figur 6a - eine perspektivische Darstellung eines Abschnitts eines weiteren Ausführungsbeispiels des erfindungsgemäßen Instruments mit dessen distalem Ende,

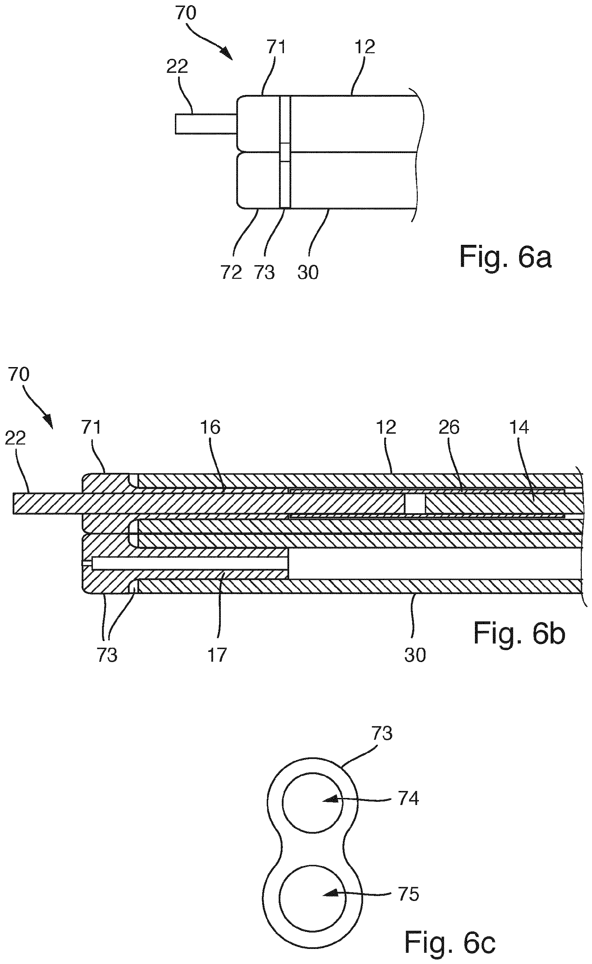

Figur 6a - eine Längsschnittdarstellung eines Abschnitts des Instruments gemäß Figur 6a mit dessen distalem Ende,
Figur 6c - ein Beispiel eines dritten Teils der Verbindungseinrichtung zur Verbindung der Schläuche des Ausführungsbeispiels gemäß Figuren 6a und 6b,

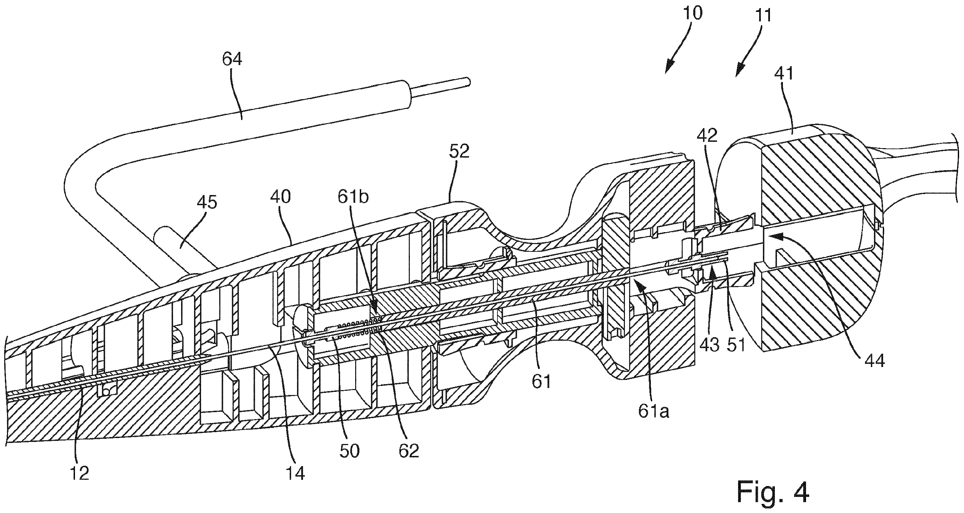

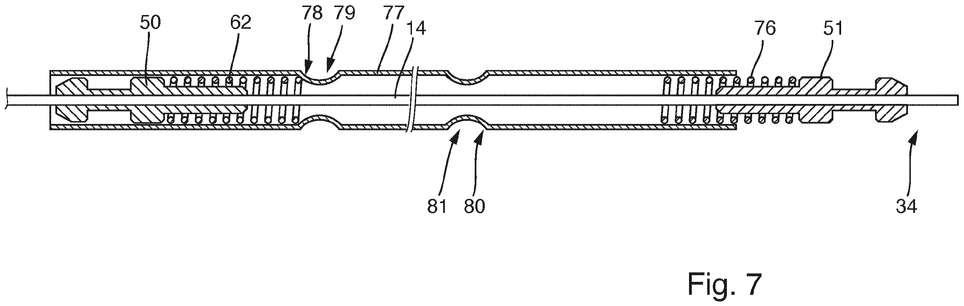

Figur 7 - eine Gruppe mit einem alternativen Schubkraftübertragungselement, welches in dem Ausführungsbeispiel gemäß Figur 4 eingesetzt werden kann,

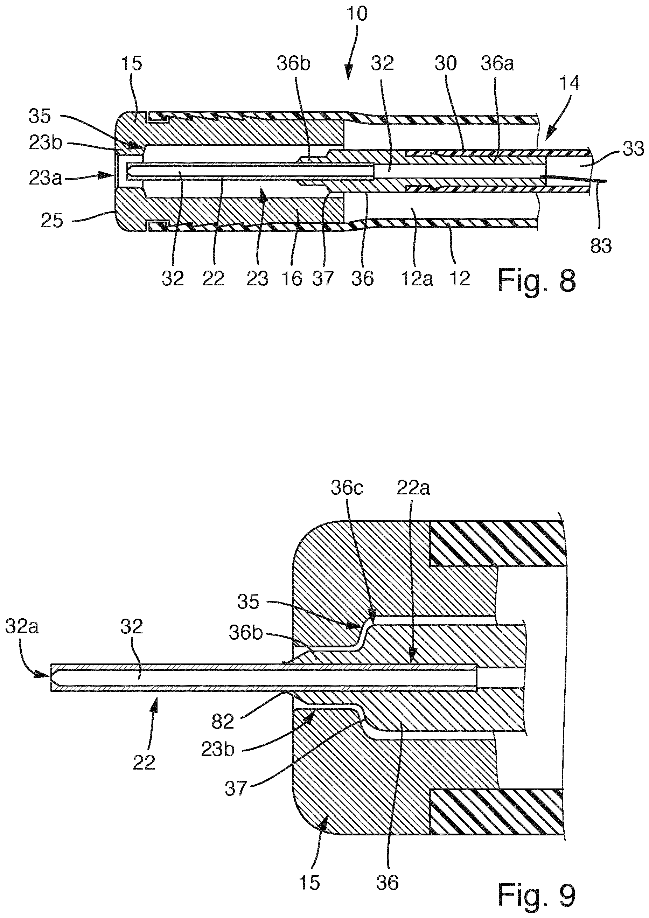

Figur 8 - ein Instrument in Koaxialbauweise, in längs geschnittener ausschnittsweiser Ansicht,
Figur 9 - das distale Ende des Instruments nach Figur 8

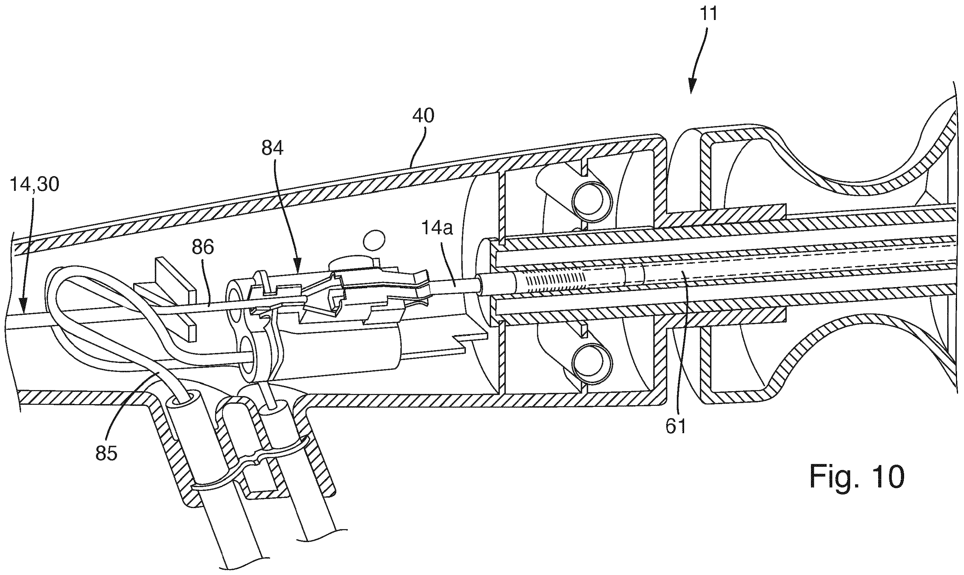

Figur 10 - ein Handgriff zur Bedienung des Instruments nach den Figuren 8 und 9.

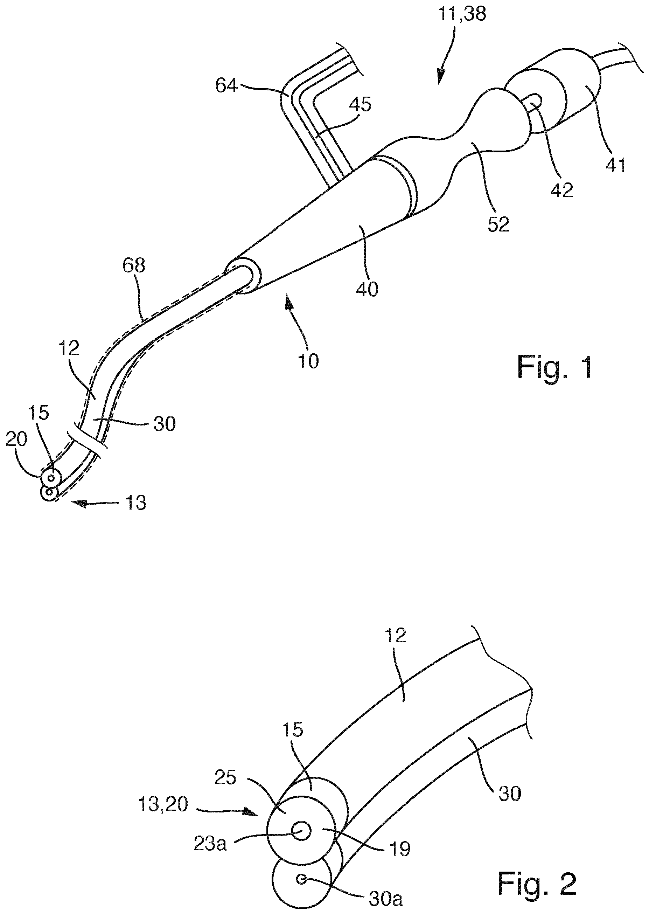

Im folgenden beschriebene Ausführungsformen erfindungsgemäßer Instrumente 10, wie eines beispielhaft in den Figuren 1 bis 4 dargestellt ist, weisen bevorzugt ein Handgriffteil 11 (Handgriff) und einen flexiblen Instrumentenschlauch 12 auf, der sich von dem Handgriffteil 11 zum distalen Ende 13 des Instruments 10 erstreckt.

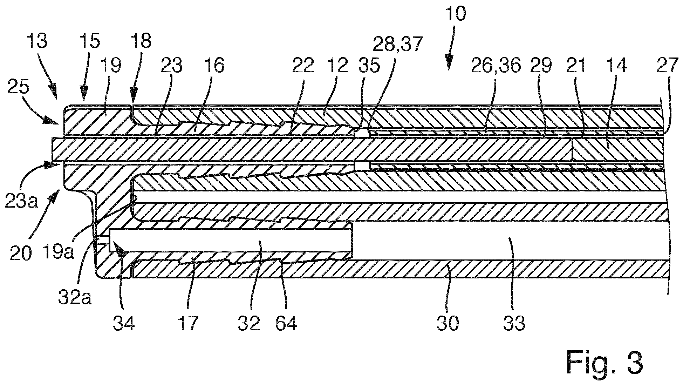

Wie in Figur 3 veranschaulicht erstreckt sich in dem Instrumentenschlauch 12 aus elektrisch isolierendem Material ein längsverschiebbares Schubelement 14. Das Schubelement 14 ist vorzugsweise elektrisch leitfähig oder enthält einen elektrischen Leiter, um die Elektrode 22 mit elektrischer Leistung zu versorgen. Das Schubelement 14 kann ein Metall-Draht, z.B. aus Nitinol sein.

Der Außendurchmesser des Schubelements 14 ist kleiner als der Innendurchmesser des Instrumentenschlauchs 12 und mit Querspiel in dem Instrumentenschlauch 12 längsbeweglich geführt.

Ein als Endstück dienender Isolatorkörper 15 weist zwei zueinander parallel orientierte röhrchenartige Fortsätze 16, 17 auf. Ein distales Ende 18 des Instrumentenschlauchs 12 sitzt auf dem Fortsatz 16 des Verbindungselements 15. Das Verbindungselement 15 und der Instrumentenschlauch 12 bilden gemeinsam eine Isolatoranordnung, deren Kopfteil 19 das distale Ende derselben ist. Das Verbindungselement 15 und somit der Kopfteil 19 und die Fortsätze 16, 17 sind bevorzugt nahtlos einstückig ausgebildet. Das Verbindungselement 15 kann beispielsweise aus Keramik oder thermisch stabilen Kunststoff gebildet sein. Der Instrumentenschlauch 12 erstreckt sich bis zu der in proximale Richtung weisenden Rückseite 19a des Kopfteils 19.

Das erfindungsgemäße Instrument 10 kann ein monopolares Instrument sein und entsprechend nur eine Elektrode 22 aufweisen. Das Schubelement 14 weist ein distales Ende 21 auf, das mit der z.B. nadelförmigen Elektrode 22 gekoppelt ist. Die Elektrode 22 ist in einem sich durch das distale Ende 18 des Instrumentenschlauches 12 sowie den Fortsatz 16 und das Kopfteil 19 erstreckenden Kanal 23 längs verschiebbar geführt. So lässt sich die Elektrode 22 längs aus einem Ausgang 23a des Kanals 23 an der in distale Richtung weisenden Stirnseite 25 heraus verschieben. Die beispielsweise aus Wolfram bestehende Nadelelektrode 22 kann mittels einer Hülse 26, insbesondere Edelstahlhülse, mit dem Schubelement 14 mechanisch und bevorzugt auch elektrisch gekoppelt sein, um eine elektrische Verbindung zwischen dem Schubelement 14 und der Elektrode 22 herzustellen.

Das distale Ende 21 des Schubelements 14 ist in einem proximalen Ende 27 der Hülse 26 angeordnet. Das proximale Ende 29 der Nadelelektrode 22 ist in einem distalen Ende 28 der Hülse 26 angeordnet. Das distale Ende 21 des Schubelements 14 und das proximale Ende 29 der Elektrode 22 können mit der Hülse 26 verschweißt sein, um das Schubelement 14 mit der Elektrode 22 zumindest mechanisch, vorzugsweise auch elektrisch, wirksam zu koppeln.

Die Edelstahlhülse 26 versteift die Anordnung des distalen Endes 21 des Schubelements 14 und des proximalen Endes 29 der Elektrode 22. Deshalb endet die Edelstahlhülse 26 proximal benachbart zu dem distalen Ende 21 des Schubelements 14, um die Flexibilität des Instruments 10 weitgehend unbeeinflusst zu lassen.

Der zweite Fortsatz 17 ist benachbart zu dem ersten Fortsatz 16 an der Seite des Verbindungselements 15 ausgebildet. Beide Fortsätze 16, 17 erstrecken sich von dem Kopfteil 19 des Verbindungselements 15 in proximaler Richtung. Es handelt sich bei dem beispielhaften Instrument 10 um ein zweilumiges Instrument 10. Das erste Lumen führt durch den Instrumentenschlauch 12. Auf dem zweiten Fortsatz 17 sitzt ein Zusatzschlauch 30 mit einem zweiten Lumen. Dieses kann ein Fluid, insbesondere eine Flüssigkeit, beispielsweise NaCl-Lösung, zu dem distalen Ende 13 des Instruments 10 führen.

Der auf dem ersten Fortsatz 16 sitzende Instrumentenschlauch 12 kann reibschlüssig oder mittels Formschlussstrukturen gehalten sein. Ebenso kann der auf dem zweiten Fortsatz 17 sitzende Zusatzschlauch 30 reibschlüssig oder mittels Formschlussstrukturen 64 gehalten sein, die auf der äußeren Oberfläche des zweiten Fortsatzes 17 und der Innenseite des distalen Endabschnitts 31 des weiteren Zusatzschlauchs 30 beruhen. Die Verbindung zwischen dem Zusatzschlauch 30 und dem zweiten Fortsatz 17 ist vorzugsweise fluiddicht.

Der zweite Fortsatz 17 kann einen Kanal 32 umschließen, welcher mit einem Kanal 33, den der Zusatzschlauch 30 umgrenzt, fluidisch verbunden ist. Der Kanal 32 kann an dem distalen Ende einen Düsenabschnitt aufweisen, in welchem der Strömungsquerschnitt verengt ist. Der Kanal 32 mündet in einem Ausgang 32a, der ebenso wie der Ausgang 23a für die Elektrode 22 an der distalen Stirnseite 25 des Verbindungselements 15 angeordnet ist.

Der erste Fortsatz 17, auf welchem der Instrumentenschlauch 12 steckt, bildet mit seiner proximalen Stirnfläche 35 einen Anschlag, um die Position der Elektrode 22 und/oder des distalen Endes 21 des Schubelements 14 bezüglich des distalen Endes der Isolatoranordnung und/oder des distalen Endes des Instrumentenschlauchs 12 festzulegen. Die Hülse 26 bildet ein Gegenanschlagelement 36, wobei die distale Stirnseite 37 der Hülse 26 einen Gegenanschlag bildet, um die Position der Elektrode 22 und/oder des distalen Endes 21 des Schubelements 14 bezüglich des distalen Endes 20 der Isolatoranordnung und/oder des distalen Endes des Schlauches 18 festzulegen. In anderen Ausführungsformen kann der Gegenanschlag beispielsweise von einer Verdickung der Elektrode 22 benachbart zu deren proximalem Ende gebildet sein.

Das aus Figur 4 ersichtliche Handgriffteil 11 am proximalen Ende 38 des Instruments 10 weist einen distalen Abschnitt 40, einen proximalen Abschnitt 41 und einen Schaftabschnitt 42 auf, der den distalen Abschnitt 40 und dem proximalen Abschnitt 41 des Handgriffteils 11 miteinander starr verbindet. Der proximale Endabschnitt 43 des Schubelements 14 erstreckt sich durch den distalen Abschnitt 40 und in den Schaftabschnitt 42 hinein bis vor dessen proximales Ende 44. Der Instrumentenschlauch 12, durch welchen sich das Schubelement 14 erstreckt, erstreckt sich in den distalen Abschnitt 40 des Handgriffteils 11 hinein und ist dort fixiert. Das Schubelement 14 kann mittels eines Schleifkontakts mit einer elektrischen Zuleitung 45 elektrisch verbunden sein (Schleifkontakt nicht dargestellt).

An dem Schubelement 14 ist ein erstes Kopplungselement 50 fixiert. Im dargestellten Ausführungsbeispiel umschließt das erste Kopplungselement 50 das Schubelement 14. An dem proximalen Ende 44 des Schaftabschnitts 42 ist das Schubelement 14 mit einem zweiten Kopplungselement 51 gekoppelt. Auf dem Schaftabschnitt 42 ist längsbeweglich ein mit den Fingern betätigbares Bedienteil 52 angeordnet. Dieses Bedienteil 52 kann von einer ersten Stellung (nicht dargestellt), in welcher das Bedienteil 52 an dem proximalen Abschnitt 41 angeordnet ist, in eine zweite Stellung (in Figur 4 dargestellt), in welcher das Bedienteil 52 an dem distalen Abschnitt 40 angeordnet ist, vorgeschoben werden, um die Elektrode 22 von einer zurückgezogenen Position in eine bestimmte vorgeschobene Position vorzuschieben, in welcher die Elektrodenspitze 60 einen definierten Abstand zur Stirnseite 25 des Verbindungselements 15 und damit zum distalen Ende 20 der Isolatoranordnung aufweist.

Wie in Figur 4 weiter veranschaulicht, ist das Bedienteil 52 dazu mit einem Schubkraftübertragungselement 61 gekoppelt, welches beispielsweise röhrenförmig ist, durch welches sich im Ausführungsbeispiel das Schubelement 14 erstreckt, welches Schubkraftübertragungselement 61 sich im Ausführungsbeispiel in dem Schaftabschnitt 42 längs des Schaftabschnitts 42 verschiebbar erstreckt. Zwischen dem Schubkraftübertragungselement 61 und dem ersten Kopplungselement 50 ist eine Druckfeder 62 angeordnet, durch welche sich das Schubelement 14 erstreckt.

Das Bedienteil 52 kann in der ersten Stellung und/oder in der zweiten Stellung vorzugsweise formschlüssig festgesetzt, insbesondere verrastet, werden.

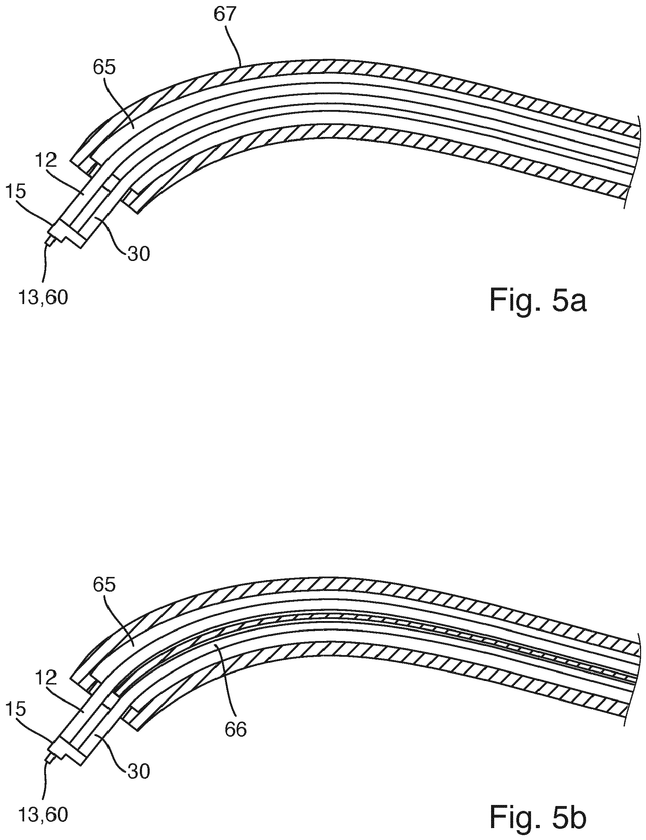

Der Instrumentenschlauch 12 und der Zusatzschlauch 30 können gemeinsam durch einen Arbeitskanal 65 (s. Figur 5a) oder, jeder für sich, durch zwei Arbeitskanäle 65, 66 eines Endoskops 67 (s. Figur 5b) geführt sein bzw. geführt werden.

Mit dem erfindungsgemäßen Instrument 10 kann wie folgt gearbeitet werden:
Anders als in Figur 4 dargestellt, wird von einer Anordnung des Bedienteils 52 am Handgriffteil 11 des Instruments 10 in der ersten Stellung ausgegangen. Die Elektrode 22 ist entsprechend in einer zurückgezogenen Stellung. Das distale Ende der Elektrode 22 kann auch in dieser Stellung aus dem distalen Ende des Verbindungselements 15 hervorschauen. Bevorzugt aber ist die (aus Figur 5a ersichtliche) Elektrodenspitze 60 proximal gegenüber dem Ausgang 23a der Isolatoranordnung (dem Ausgang 23a des Verbindungselements 15) zurückgezogen, durch welchen die Elektrode 22 geschoben werden kann, oder schließt mit dem Ausgang des Verbindungselements 15 ab.

Das Instrument 10 kann sich durch ein Endoskop erstrecken (nicht dargestellt), wobei das Verbindungselement 15 außerhalb des Endoskops angeordnet ist. Der Anwender möchte die Elektrodenspitze 60 nun vorschieben, und zwar in eine Position mit einem definierten Abstand der Elektrodenspitze 60 zum Ausgang 23a der Isolatoranordnung. Dazu muss der Anwender gegebenenfalls die Verrastung des Bedienteils 52 in der ersten Stellung lösen und das Bedienteil 52 in distaler Richtung relativ zu dem distalen Abschnitt 40, dem Schaftabschnitt 42 und dem proximalen Abschnitt 41 vorschieben, wobei diese Vorschubbewegung auf das Schubkraftübertragungselement 61 übertragen wird. Über das Schubkraftübertragungselement 61 wird durch das Vorschieben des Bedienteils 52 schließlich eine Kraft auf die Druckfeder 62 in Längsrichtung der Druckfeder 62 ausgeübt, welche die Kraft auf das erste Kopplungselement 50 und darüber auf das Schubelement 14 überträgt, so dass das Schubelement 14 in distale Richtung vorgeschoben wird und damit auch die Elektrode 22 in distale Richtung vorschiebt. Das Vorschieben ist vorzugsweise weitgehend reibungskräftefrei möglich. Auf ein im Stand der Technik genutztes Mittel zum Verhindern einer ungewollten Bewegung der Elektrode in proximale Richtung beim Krümmen oder Abknicken des Schlauchs durch Reibung kann verzichtet werden.

Wenn der Gegenanschlag 37 des Gegenanschlagelements 36 mit dem Anschlag 35 an dem Verbindungselement 15 in Anlage kommt, hat das Bedienteil 52 die zweite Stellung noch nicht erreicht. Der Anwender schiebt das zweite Bedienteil 52 nun weiter in distale Richtung und staucht dabei die Druckfeder 62 in Richtung des Kraftflusses von dem Bedienteil 52 zu dem Gegenanschlagelement 36, um das Gegenanschlagelement 36 gegen das Anschlagelement 15 vorzuspannen. Schließlich erreicht der Anwender mit dem Bedienteil 52 die zweite Stellung, in welcher das Bedienteil 52 verrastet werden kann. Die Elektrode 22 hat, wenn der Gegenanschlag 37 mit dem Anschlag 35 in Anlage geraten ist, ihre definierte Position erreicht. Die gestauchte Feder 62 bildet eine Kraftreserve bzw. sorgt für eine Längenreserve, welche Kraftreserve dazu dient, den Gegenanschlag 37 auch dann in Anschlag zum dem Anschlag 35 zu halten, wenn das Endoskop und/oder der Instrumentenschlauch 12 mehr oder weniger stark abgewinkelt oder gekrümmt werden. Die Anordnung mit dem gespannten Federelement 62 bildet einen Permanentspanner für den Gegenanschlag 37 gegen den Anschlag 35. Bevorzugt wird in allen mit dem Instrument 10 erreichbaren chirurgischen Einsatzfällen das Gegenanschlagelement 36 in Anlage mit dem Anschlagelement 15 und somit die Position der Elektrodenspitze 60 relativ zu dem distalen Ende 20 der Isolatoranordnung gehalten. Mittels der vorgespannten Feder 62 wird ein Längenausgleich im Instrument 10 erbracht und die Elektrode in der ausgeschobenen Position gehalten. Das elastische Vorspannen des Gegenanschlags 37 in distaler Richtung erfolgt vorzugsweise nicht oder nicht allein durch seitliches, insbesondere zufälliges, Ausweichen oder Schlängeln des Schubelements 14 auf Grund der mittels des Bedienelements 52 eingeleiteten Schubkraft. Vielmehr bildet die Druckfeder 62 eine gesonderte elastisch verformbare Einrichtung, welche dazu eingerichtet und bestimmt ist, in elastisch verformtem Zustand eine Längenreserve zu bilden. Alternativ oder zusätzlich zu einem Federelement 62, insbesondere einer Druckfeder, kann die Einrichtung durch einen Abschnitt des Schubelements 14 gebildet sein (nicht dargestellt), welcher dazu eingerichtet, beispielsweise geformt, und bestimmt ist, beim Vorschieben der Elektrode 22 in distale Richtung elastisch in Vorschubrichtung in der Länge gestaucht zu werden. Der Abschnitt kann beispielsweise schraubenförmig gewunden sein. Der Abschnitt ist vorzugsweise in der Länge auf einen bestimmten Unterabschnitt des Schubelements begrenzt. Gleich ob die elastisch verformbare Einrichtung durch ein Federelement 62 und/oder einen elastisch verformbaren Abschnitt des Schubelements 14 gebildet ist, ist die Einrichtung vorzugsweise im Handgriffteil 11 des Instruments 10 angeordnet.

Der Anwender kann über die Zuleitung 45, den Schleifkontakt und das Schubelement 14 die Elektrode mit HF-Leistung beaufschlagen, um eine Behandlung am Patienten durchzuführen. Beispielsweise kann der Anwender mit der Elektrode Gewebe des Patienten schneiden. Alternativ oder zusätzlich kann der Anwender über eine Fluidzuleitung 63, welche mit dem Zusatzschlauch 30 verbunden ist, Fluid, insbesondere NaCl-Lösung, in Zusatzschlauch 30 und in den Kanal 32 an dem distalen Ende des Verbindungselements 15 und aus diesem heraus führen. Die Druckfeder 62 kann im eingezogenen Zustand der Elektrode 22 (Bedienteil 52 in erster Stellung) entspannt sein und im ausgeschobenen Zustand stark zusammengedrückt sein oder die Druckfeder 62 kann auch in der ersten Stellung des Bedienteils 52 bereits in distale Richtung elastisch verformt (vorgespannt) sein.

Um die Elektrode 22 zurückzubewegen, kann der Anwender die Verrastung des Bedienteils 52 lösen, wonach sich die Feder 62 entspannt und die Elektrode 22 über das Schubelement 14, das mit der Elektrode 22 über die Hülse 26 verbunden ist, zurückzieht. Der Bediener kann das Bedienteil 52 in die zweite Stellung zurückziehen, in der Elektrode 22 weitest möglich zurückgezogen ist und das Bedienteil 52 in der zweiten Stellung verrasten.

Wie in Figur 1 mit gestrichelten Linien angedeutet, können der Instrumentenschlauch 12 und der weitere Schlauch in einem Hohlraum einer Hülle 68 angeordnet sein. Die Hülle 68 kann sich von dem Handgriffteil 11 bis zu dem Verbindungselement 15 erstrecken. Die Hülle 68 kann ein einstückiger oder einteiliger Schrumpfschlauch sein, welcher sich von dem Handgriffteil 11 bis zu dem Verbindungselement 15 erstreckt und um den Instrumentenschlauch 12 und den Zusatzschlauch 30 aufgeschrumpft ist. Alternativ kann die Hülle 68 aus einer Anzahl an Schrumpfschlauchabschnitten zusammengesetzt sein, welche über die gesamte Länge des Instrumentenschlauchs 12 und des Zusatzschlauchs 30 verteilt und aufgeschrumpft sind. Dies hat gegenüber dem Schrumpfschlauch mit einem durchgehenden Abschnitt über die gesamte Länge der Schläuche 12, 30 den Vorteil, dass die Schläuche 12, 30 mit der so ausgebildeten Umhüllung flexibler sind.

Figur 6a bis 6b stellen ausschnittsweise beispielhafte Ansichten eines Instruments 10 gemäß einer weiteren Ausführungsform dar. Zur Verbindung des Instrumentenschlauchs 12 und des Zusatzschlauchs 30 ist eine Verbindungseinrichtung 70 vorgesehen. Zu dieser gehören ein erstes Teil 71, welches einen Fortsatz 16 aufweist, und ein zweites Teil 72, welches einen weiteren Fortsatz 17, aufweist, wobei die beiden Teile 71, 72 gesonderte Teile z.B. aus Keramik sind. Ein drittes Teil 73 der Verbindungseinrichtung 70, welches von dem ersten Teil 71 und dem zweiten Teil 72 gesondert ist, dient der Verbindung der beiden Teile 71, 72 miteinander und damit der Verbindung des Instrumentenschlauchs 12 und des Zusatzschlauchs 30. Das dritte Teil 73 kann beispielsweise ein flaches Metallteil sein. Das dritte Teil 73 weist wenigstens eine Öffnung, bevorzugt, wie in Figur 6c dargestellt, je eine Öffnung 74, 75 für das erste Teil 71 und das zweite Teil 72 auf. Das dritte Teil 73 kann die Form einer Acht aufweisen. Um eine Verbindung zwischen dem Instrumentenschlauch 12 und dem Zusatzschlauch 30 herzustellen, werden der Fortsatz 16 des ersten Teils 71 und der Fortsatz 17 des zweiten Teils 72 in die Öffnungen 74, 75 des dritten Teils 73 gesteckt. Dann werden der Instrumentenschlauch 12 und der Zusatzschlauch 30 auf die Fortsätze 16, 17 des ersten Teils 71 bzw. des zweiten Teils 72 geschoben. Die Schläuche 12, 30 können gegen das dritte Teil 73 geschoben werden. Die Verbindung zwischen dem Instrumentenschlauch 12 und dem Zusatzschlauch 30 ist damit hergestellt. Die Außenkontur des dritten Teils 73 kann den Schlauchkonturen folgen. Die proximale Stirnfläche 35 des Fortsatzes 16 des ersten Teils 71 bildet einen Anschlag für die Stirnseite 37 am distalen Ende 28 der Hülse 26 wie und zu welchem Zweck im Zusammenhang mit der Ausführungsform gemäß Figur 1 bis 4 beschrieben ist.

In der in Figur 4 dargestellten Ausführungsform ist es möglich, das Bedienteil 52 so weit zurückzuziehen, bis dieses an den proximalen Abschnitt 41 stößt. Im Zuge der Bewegung wird die proximale Stirnfläche 61a des Schubkraftübertragungselements 61 gegen das zweite Kopplungselement 51 gedrängt, so dass die Nadelelektrode 22 entsprechend weit in den Kanal 23 des Verbindungselements 15 zurückgezogen wird. Das Instrument 10 kann eine Elektrode aufweisen, die sich nicht vollständig in den Kanal 23 zurückziehen lässt, weil die Abmessung der Elektrode an ihrem distalen Ende zu groß für den Kanal 23 ist. Z.B. kann es sich bei der Elektrode um eine L-förmige Elektrode oder eine nadelförmige Elektrode mit einem Ring um die Nadel handeln. Wird nun versucht, das Bedienteil 52 weiter in proximale Richtung zu bewegen, kann dabei eventuell eine zu hohe Kraft von dem Bedienteil 52 auf die Elektrode übertragen werden, welche dabei Schaden nehmen kann. Ein zwischen der proximalen Stirnfläche 61a des Schubkraftübertragungselements 61 und dem zweiten Kopplungselement 51 angeordnetes weiteres Federelement 76 verhindert dies. Das zweite Kopplungselement 51 ist dann entsprechend in Richtung proximales Ende des Schubelements 14 versetzt angeordnet. Beispielsweise kann sich das Schubelement 14 durch eine weitere Druckfeder 76 erstrecken, welche zwischen dem Schubkraftübertragungselement 61 und dem zweiten Kopplungselement 51 angeordnet ist (in Figur 4 nicht dargestellt). Bei einer Bewegung des Bedienteils 52 zum Zurückziehen des Schubelements 14 bzw. der Elektrode, wird das weitere Federelement 76 in proximaler Richtung gegen das zweite, mit dem Schubelement 14 gekoppelte Kopplungselement 51 geschoben, um die Bewegung des Bedienteils 52 zum Zurückziehen der Elektrode auf das Schubelement 14 zu übertragen. Wenn sich die Elektrode und/oder das Schubelement 14 nicht mehr weiter in den Kanal und/oder den Schlauch zurückziehen lassen, wird bei weiterer Bewegung des Bedienteils 52 das zweite Federelement 76 elastisch verformt. Dies führt zu einer Begrenzung der Kraft auf die beispielsweise L-förmige Elektrode bzw. das Schubelement 14.

Ein weiteres dem vorstehend beschriebenen Zweck dienendes Federelement 76 ist in Figur 7 dargestellt. Figur 7 zeigt auch ein alternatives Schubkraftübertragungselement 77, welches anstelle des Schubkraftübertragungselements 61 gemäß Figur 4 in dem Instrument 10 verwendet werden kann. Das Schubkraftübertragungselement 77 gemäß Figur 7 ist dazu eingerichtet, ein seitliches (quer zur Längserstreckungsrichtung des Schubelements 14) Ausweichen der in dem Schubkraftübertragungselement 77 angeordneten Elemente zu begrenzen. Das Schubkraftübertragungselement 77 umschließt das erste Kopplungselement 50 und die Druckfeder 62 und begrenzt ein Ausweichen der Druckfeder 62, des ersten Kopplungselements 50 und/oder des Schubelements 14. Ebenso umschließt das Schubkraftübertragungselement 77 das zweite Kopplungselement 51 und die weitere Druckfeder 76 und begrenzt ein Ausweichen der weiteren Druckfeder 76, des zweiten Kopplungselements 51 und/oder des Schubelements 14 beim Zurückziehen der Elektrode 22. Das Schubkraftübertragungselement 77 kann als dünnwandiges Rohr 77, insbesondere Edelstahlrohr, ausgebildet sein. Die Enden des Rohres umschließen die Druckfedern 62, 76 und die Kopplungselemente 50, 51. Das Schubkraftübertragungselement 77 weist einen ersten Anschlagabschnitt 78 auf, welcher im dargestellten Ausführungsbeispiel durch eine Rille 79 gebildet ist. Der Anschlagabschnitt 78 wird beim Vorschieben des Schubkraftübertragungselements 77 gegen die Druckfeder 62 gedrängt. So wird die Kraft von dem mit dem Schubkraftübertragungselement 77 gekoppelten Bedienteil 52 in distaler Richtung über das erste Kopplungselement 50 auf das Schubelement 14 übertragen. Der erste Anschlagabschnitt 78 des Schubkraftübertragungselements 77 übernimmt insofern die Funktion der distalen Stirnseite 61b des Schubkraftübertragungselements 61 aus Figur 4. Das Schubkraftübertragungselement 77 weist zudem einen zweiten Anschlagabschnitt 80 auf, welcher im dargestellten Ausführungsbeispiel durch eine zweite Rille 81 gebildet ist. Der Anschlagabschnitt 80 wird beim Zurückbewegen des Schubkraftübertragungselements 77 gegen die weitere Druckfeder 76 gedrängt. Diese überträgt die Kraft von dem Bedienteil 52 auf das Schubelement 14, um die Elektrode 22 zurück zu ziehen. Der zweite Anschlagabschnitt 80 übernimmt insofern die Funktion der proximalen Stirnseite 61a des Schubkraftübertragungselements 61 aus Figur 4. Alternativ zu Ausführungsformen mit einem Federelement 62 zum Längsausgleich in distale Richtung und einem weiteren Federelement 76 zum Längenausgleich in proximale Richtung können entsprechende Längenausgleiche auch mit nur einer elastisch verformbaren Einrichtung, insbesondere einem Federelement, geschaffen sein, die dazu eingerichtet und angeordnet ist, elastisch in zwei Richtungen verformt zu werden. Außerdem kann das Schubelement 14 zugelastisch ausgebildet sein und als Zugfederelement dienen.

Figur 8 zeigt eine abgewandelte Ausführungsform des Instruments 10. Für diese Ausführungsform gilt die vorstehende Erläuterung unter Zugrundelegung gleicher Bezugszeichen sowohl hinsichtlich Struktur als auch hinsichtlich Funktion unter Berücksichtigung der nachfolgenden Erläuterungen entsprechend.

Abweichend von dem vorig beschriebenen Instrument nach Figur 1 bis 7 ist das in den Figuren 8 und 9 veranschaulichte Instrument 10 ein koaxial aufgebautes Instrument mit einer hohl ausgebildeten Elektrode 22, die an ihrem distalen Ende eine Austrittsöffnung 32a aufweist. Der sich durch die Elektrode 25 erstreckende Kanal steht mit dem Zusatzschlauch 30 in Fluidverbindung, der sich durch das Lumen 12a bis zu dem Handgriffteil 11 erstreckt. Der Instrumentenschlauch 30 ist einigermaßen drucksteif, sodass er zwar biegsam, dabei aber durchaus zur Übertragung von Druckkräften geeignet ist. Zur Verbindung zwischen dem Instrumentenschlauch 30 und der Elektrode 32 kann das hier als Elektrodensockel ausgebildete Gegenanschlagelement 36 dienen, in dem, wie Figur 9 zeigt, das proximale Ende 22a der Elektrode 22 gehalten ist. Das Gegenanschlagelement 36 weist einen sich in proximaler Richtung in den Zusatzschlauch 30 hinein erstreckenden Schaft 36 auf, der mit dem Zusatzschlauch 30 zug- und druckfest verbunden ist und durch den sich ein den Kanal 33 mit dem Kanal 32 verbindender Durchgang erstreckt.

Das Anschlagelement 35 weist in seinem Kanal 23 eine dem Gegenanschlagelement 36 zugewandte Ringschulter auf, die als Anschlag 15 für das Gegenanschlagelement 36 dient. Von der Ringschulter ausgehend verengt sich der Kanal 23 auf einen engeren Abschnitt 23b.

Das Gegenanschlagelement 36 weist einen durch den verengten Abschnitt 23b passenden Fortsatz 36b auf, dessen Außendurchmesser geringer ist als der Außendurchmesser des übrigen Gegenanschlagelements 36. Das Gegenanschlagelement 36 weist entsprechend eine Ringschulter 36c auf, die mit dem Anschlag 35 in Anlage kommt, wenn die Elektrode 22 ganz in expandierten Stellung steht. Zur Verbindung der Elektrode 22 mit dem Gegenanschlagelement 36 können ein oder mehreren Schweißverbindungen 82 vorgesehen sein, die an dem in expandierter Position die distale Stirnseite 25 überragenden Teil des Abschnitts 36b des Gegenanschlagelements 36 vorgesehen ist bzw. sind.

Der Instrumentenschlauch 12 und der Zusatzschlauch 30 können aus einem Kunststoff ausgebildet sein. Zur Stromversorgung der Elektrode 22 kann ein sich durch das den Kanal 33 des Zusatzschlauchs 30 erstreckender elektrischer Leiter 83 vorgesehen sein. Dieser kann mit dem Schaft 36a des Gegenanschlagelements 36 beispielsweise durch Schweißen elektrisch verbunden sein. Es ist auch möglich, den elektrischen Leiter 83 in den Kunststoff des Zusatzschlauchs 30 einzuarbeiten oder den elektrischen Leiter 83 in dem Lumen 12a des Instrumentenschlauchs 12 zu führen. In allen Fällen der koaxialen Instrumentengestaltung nach Figur 8 kann der elektrische Leiter 83 entweder selbst zug- und drucksteif sein und somit einen Teil des Schubelements 14 bilden. Alternativ kann der biegeschlaff ausgebildet sein, sodass das Schubelement 14 allein durch den Zusatzschlauch 30 gebildet ist.

Figur 10 veranschaulicht ein etwas abgewandeltes Handgriffteil 11 zur Bedienung des Instruments 10 nach den Figuren 8 und 9. Hinsichtlich der Betätigung des Instruments gilt die vorige Beschreibung mit folgender Ergänzung entsprechend:

Der als Schubelement 14 dienende Zusatzschlauch 30 führt zu einer Fluidweiche 84, die axial beweglich ist in dem als Gehäuse ausgebildeten distalen Abschnitt 40 des Handgriffteils 11 angeordnet ist. An die Fluidweiche 84 ist ein Fluidzufuhrschlauch 85 angeschlossen, der über einen in der Fluidweiche 84 ausgebildeten Kanal fluidmäßig mit dem Zusatzschlauch 30 verbunden ist. Außerdem stellt die Fluidweiche 34 eine mechanische zug- und druckfeste Verbindung zwischen dem Zusatzschlauch 30 und dem proximalen Schubelementabschnitt 14a her, der sich als zug- und drucksteifer Stift oder Draht in das Schubkraftübertragungselement 61 hinein erstreckt. Die Fluidweiche 84 besteht vorzugsweise aus Metall. Ein elektrischer flexibler Zuleitungsdraht 86 kann außen an die Fluidweiche 34 angeschlossen sein. Der durch den Zusatzschlauch 14 geführte Leiter 83 kann in der Fluidweiche 84 elektrisch mit dieser kontaktiert sein. Ansonsten gilt die im Zusammenhang mit Figur 4 gegebene Beschreibung entsprechend.

Das elektrochirurgische Instrument 10 weist eine Elektrode 22 und eine Isolatoranordnung auf. Diese umfasst einen Instrumentenschlauch 12 und eine an seinem distalen Ende angeordnete Isolatoranordnung. In Instrumentenschlauch 12 ist ein längliches Schubelement 14 zum Festlegen der Position der Elektrode 22 relativ zum distalen Ende der Isolatoranordnung angeordnet. Das Instrument 10 weist einen Anschlag 35 und einen Gegenanschlag 37 auf, wobei der Gegenanschlag der Elektrode 22 und/oder dem Schubelements 14 zugeordnet ist. Eine elastisch verformbare Einrichtung 62 ist dazu bestimmt, den Gegenanschlag 37 in distale Richtung gegen den Anschlag 35 vorzuspannen, um beim Abknicken oder Krümmen des Instrumentenschlauchs 12 eine Bewegung der Elektrode 22 in proximale Richtung relativ zu dem distalen Ende 20 der Isolatoranordnung und/oder des Instrumentenschlauchs 12 zu verhindern.

**Bezugszeichenliste:**

| | |
|---|---|
| 10 | Instrument |
| 11 | Handgriff teil |
| 12 | Instrumentenschlauch |
| 12a | Lumen des Instrumentenschlauchs |
| 13 | Distales Ende des Instruments |
| 14 | Schubelement |
| 14a | Schubelementabschnitt |
| 15 | Verbindungselement/Anschlagelement |
| 16 | Erster Fortsatz/Fortsatz |
| 17 | Zweiter Fortsatz/weiterer Fortsatz |
| 18 | distales Ende des Schlauches |
| 19 | Kopfteil |
| 19a | Rückseite |
| 20 | Distales Ende Isolatoranordnung |
| 21 | Distales Ende des Schubelements |
| 22 | Nadelelektrode |
| 22a | Proximales Ende der Elektrode 22 |
| 23 | Kanal |
| 23a | Ausgang |
| 23b | Verengter Abschnitt des Kanals 23 |
| 25 | Stirnseite |
| 26 | Hülse |
| 27 | Proximales Ende der Hülse |
| 28 | Distales Ende der Hülse |
| 29 | Proximales Ende der Nadelelektrode |
| 30 | Zusatzschlauch |
| 31 | Distaler Endabschnitt |
| 32 | Kanal |
| 32a | Ausgang, Austrittsöffnung |
| 33 | Kanal |
| 34 | Düsenabschnitt |
| 35 | Proximale Stirnfläche/Anschlag |
| 36 | Gegenanschlagelement |
| 36a | Schaft |
| 36b | Abschnitt des Gegenanschlagelements 36 |
| 36c | Ringschulter an dem Gegenanschlagelement 36 |
| 37 | Distale Stirnseite/Gegenanschlag |
| 38 | Proximales Ende des Instruments |
| 40 | Distaler Abschnitt |
| 41 | Proximaler Abschnitt |
| 42 | Schaftabschnitt |
| 43 | Proximaler Endabschnitt des Schubelements |
| 44 | Proximales Ende des Schaftabschnitts |
| 45 | Elektrische Zuleitung |
| 50 | Erstes Kopplungselement |
| 51 | Zweites Kopplungselement |
| 52 | Bedienteil |
| 60 | Elektrodenspitze |
| 61 | Schubkraftübertragungselement |
| 61a | proximale Stirnseite |
| 61b | distale Stirnseite |
| 62 | Druckfeder |
| 63 | Fluidzuleitung |
| 64 | Formschlussstruktur |
| 65 | Arbeitskanal |
| 66 | Arbeitskanal |
| 67 | Endoskop |
| 68 | Hülle |
| 70 | Verbindungseinrichtung |
| 71 | erstes Teil |
| 72 | zweite Teil |
| 73 | drittes Teil |
| 74 | Öffnung |
| 75 | Öffnung |
| 76 | weitere Druckfeder |
| 77 | Schubkraftübertragungselement/Rohr |
| 78 | Erster Anschlagabschnitt |
| 79 | Erste Rille |
| 80 | Zweiter Anschlagabschnitt |
| 81 | Zweite Rille |
| 82 | Schweißverbindung |
| 83 | Leiter |
| 84 | Fluidweiche |
| 85 | Fluid-Zufuhrschlauch |
| 86 | Zuleitungsdraht oder -Litze |
| | |
| | |
| | |
| | |
| | |
| | |

## Patentansprüche

1. Elektrochirurgisches Instrument (10) mit einer Elektrode (22), einer Isolatoranordnung (12, 15), welche einen Instrumentenschlauch (12) aufweist, in welchem ein längliches Schubelement (14) zum Festlegen der Position der Elektrode (22) relativ zum distalen Ende der Isolatoranordnung (12, 15) angeordnet ist, einem Anschlag (35), einem Gegenanschlag (37) der Elektrode (22) und/oder des Schubelements (14) sowie einer elastisch verformbaren Einrichtung (62), welche dazu angeordnet und eingerichtet ist, den Gegenanschlag (37) in distale Richtung gegen den Anschlag (35) vorzuspannen, um beim Abknicken oder Krümmen des Instrumentenschlauchs (12) eine Bewegung der Elektrode (22) in proximale Richtung relativ zu dem distalen Ende (20) der Isolatoranordnung (12, 15) und/oder des Instrumentenschlauchs (12) zu verhindern.

2. Instrument (10) nach Anspruch 1, wobei der Anschlag (35) benachbart zu dem distalen Ende (18) des Instrumentenschlauchs (12) und/oder der Isolatoranordnung (12, 15) angeordnet ist.

3. Instrument (10) nach einem der vorstehenden Ansprüche, wobei die elastisch verformbare Einrichtung (62) proximal außerhalb des Instrumentenschlauchs (12) angeordnet ist und/oder wobei das Instrument (10) einen Handgriff (11) aufweist, wobei die elastisch verformbare Einrichtung (62) in dem Handgriff (11) angeordnet ist.

4. Instrument (10) nach einem der vorstehenden Ansprüche, wobei der Anschlag (35) von einem Anschlagelement (15) gebildet ist, welches in einem distalen Endabschnitt (18) des Instrumentenschlauchs (12) steckt, gegebenenfalls form- und/oder reibschlüssig gehalten ist.

5. Instrument (10) nach einem der vorstehenden Ansprüche, wobei der Gegenanschlag (37) an der Elektrode (22) und/oder dem Schubelement (14) ausgebildet und/oder an einem Gegenanschlagelement (36) ausgebildet ist, welches mit der Elektrode (22) und/oder dem Schubelement (14) verbunden ist.

6. Instrument (10) nach einem der vorstehenden Ansprüche, wobei der Gegenanschlag (37) von einem Gegenanschlagelement (36) gebildet wird, welches ein von der Elektrode (22) und dem Schubelement (14) gesondertes Element ist und der Verbindung zwischen dem Schubelement (14) und der Elektrode (22) dient.

7. Instrument (10) nach Anspruch 5 oder 6, wobei das Gegenanschlagelement (36) einen Fortsatz (36b) aufweist, der sich bis zu einer distalen Endfläche (25) eines den Anschlag (35) aufweisenden Anschlagelements (15) oder distal darüber hinaus erstreckt.

8. Instrument (10) nach einem der vorstehenden Ansprüche, **dadurch gekennzeichnet, dass** das Schubelement (14) durch einen Zusatzschlauch (30) gebildet ist, der innerhalb des Instrumentenschlauchs (12) angeordnet ist.

9. Instrument (10) nach das Schubelement (14) ein drucksteifer Draht ist.

10. Instrument (10) nach Anspruch 8, **dadurch gekennzeichnet, dass** in oder an dem Zusatzschlauch (30) ein elektrischer Leiter (83) angeordnet ist, der mit der Elektrode (22) elektrisch verbunden ist.

11. Instrument (10) nach einem der vorstehenden Ansprüche, wobei das Gegenanschlagelement (36) eine Hülse (26) ist, in welcher einenends die Elektrode (22) und anderenends das Schubelement (14) befestigt ist.

12. Instrument (10) nach einem der vorstehenden Ansprüche, wobei das Anschlagelement (15) einen Stopperabschnitt (19) aufweist, gegen den der Schlauch (12) und/oder der Zusatzschlauch (30) geschoben sein können, um die Position des distalen Endes (18) des Instrumentenschlauchs (12) und/oder des Zusatzschlauchs (30) an dem Anschlagelement (15) festzulegen.

13. Instrument (10) nach einem der vorstehenden Ansprüche, wobei ein Fortsatz (17) des Verbindungselements (15) Formschlussstrukturen (65) aufweist, welche das Aufschieben des Zusatzschlauchs (30) auf den Fortsatz (17) mit geringerer Kraft erlauben, als das Abziehen des Zusatzschlauchs (30) von dem Fortsatz (17).

14. Instrument (10) nach einem der vorstehenden Ansprüche, wobei der Fortsatz (16) des Verbindungselements (15) für den Instrumentenschlauch (12) außen frei von Formschlussstrukturen ist.

15. Instrument (10) nach einem der vorstehenden Ansprüche, wobei der Schlauch (12) durch einen Arbeitskanal (65, 66) eines Endoskops (67) geführt ist und/oder wobei der Schlauch (12) und der Zusatzschlauchs (30) durch denselben Arbeitskanal (65) eines Endoskops (67) oder durch je einen eigenen Arbeitskanal (65, 66) geführt sind.
